# EUROPEAN PATENT APPLICATION

(11) **EP 3 753 591 A1**
(43) Date of publication of application: **23.12.2020**
(21) Application number: 20178860.1
(22) Date of filing: 09.06.2020
(51) Int. Cl.: A61M 5/142, A61M 5/32

(54) **MEDICAL DEVICE WITH ADHESIVE PATCH AND METHOD FOR MAKING SAME**

(30) Priority: 21.06.2019 US 201962864795 P
(71) Applicant: Becton, Dickinson and Company, Franklin Lakes, NJ 07417-1880 (US)
(72) Inventor: REED, Jeffrey, Franklin Lakes, New Jersey 07417-1880 (US); KNIGHT, John, Franklin Lakes, New Jersey 07417-1880 (US); TOWNSEND, Scott, Franklin Lakes, New Jersey 07417-1880 (US)
(74) Representative: dompatent von Kreisler Selting Werner - Partnerschaft von Patent- und Rechtsanwälten mbB

(57) **Abstract**

A method of manufacturing a medical device, including forming a flexible base portion to have a plurality of through-holes therethrough, placing the flexible base portion on a non-patient side of an adhesive patch with the through-holes in communication with the adhesive patch, dispensing an adhesive into the through-holes onto the adhesive patch and walls of the flexible base portion surrounding the through-holes; and curing the adhesive to form an adhesive plug mechanically bonding the flexible base portion to the adhesive patch.

## Description

### Cross-Reference to Related Application

This application claims the benefit of U.S. Provisional Application No. 62/864795, filed June 21, 2019 in the U.S. Patent and Trademark Office, the disclosure of which is incorporated herein by reference in its entirety.

### Field of the Invention

The present invention relates generally to medical devices and to methods for making such devices. In particular, but not by way of limitation, the present invention relates to an infusion set, patch pump or other on-body medical device having an adhesive patch that is secured to the medical device during manufacture of the medical device or during manufacture of a portion of the medical device.

### Background of the Invention

In the management of diabetes, insulin can be administered to a user on a continuous basis using a programmable infusion pump. The pump is small, portable and battery-operated, and can be worn or carried inconspicuously by the user. The pump is attached by a length of tubing to an infusion set, which is a disposable body-worn device having a skin-adhering base, a fluid connector, and a rigid needle or flexible catheter that conducts the insulin into the subcutaneous or intradermal layer of the user's skin. A flexible adhesive patch or pad on the bottom of the infusion set base allows for temporary skin attachment during the period (typically a few days) that the infusion set remains in place before it is removed and replaced for sanitary reasons.

Another type of insulin infusion device, known as a patch pump, has also come into use. Unlike a conventional infusion pump, a patch pump is an integrated device that combines most or all of the required components in a single housing and does not require the use of a separate infusion set or connecting tubing. A patch pump adheres directly to the skin, contains insulin in an internal reservoir, and delivers the insulin via an integrated subcutaneous catheter. As in the case of an infusion set, a patch pump typically includes a flexible adhesive patch on its bottom surface to allow for temporary skin attachment at the infusion site.

The flexible adhesive patch is typically made of a thin fibrous material, similar to a medical dressing, but with adhesive layers on both sides. On the skin contact side, an adhesive suitable for medical or surgical use is provided, and is covered by a removable backing layer or release liner made of silicone-coated kraft paper. The release liner is removed by the user before attaching the infusion set or patch pump to the skin. The skin contact adhesive must be sufficiently strong to secure the infusion set or patch pump to the skin, but must also allow for removal of the device without damaging the skin. On the device side, a different adhesive is used, one which provides for permanent attachment between the patch and the base of the infusion set or patch pump.

Some infusion sets and/or patch pumps employ a flexible material, such as thermoplastic polyurethane (TPU), for at least a part of the device to provide greater freedom of movement to the patient or to better conform to curves of a patient's body. Unfortunately, TPU and some other flexible materials sometimes have difficulty adhering to adhesive patches or pads using only an adhesive. In addition, the manufacturing process for the infusion set or patch pump may require that the adhesive patch be dispensed from a continuous roll for handling by automated equipment. This may impose additional requirements on the types of adhesives that can be used.

### Summary of Embodiments of the Invention

Accordingly, it is an aspect of the present invention to provide a medical device having a secured adhesive patch and a method of manufacturing such a medical device.

The foregoing and/or other aspects of the present invention are achieved by providing an on-body medical device, including a flexible base portion having one or more through-holes therein, an adhesive patch, the flexible base portion being disposed on a non-patient side of the adhesive patch with the through-holes in communication with the adhesive patch, and an adhesive plug cured in the one or more through-holes on the adhesive patch and walls of the flexible base portion laterally surrounding the one or more through-holes, the adhesive plug mechanically bonding the flexible base portion to the adhesive patch.

The foregoing and/or other aspects of the present invention are also achieved by providing a method of manufacturing a medical device, including forming a flexible base portion to have a plurality of through-holes therethrough, placing the flexible base portion on a non-patient side of an adhesive patch with the through-holes in communication with the adhesive patch, dispensing an adhesive into the through-holes onto the adhesive patch and walls of the flexible base portion surrounding the through-holes; and curing the adhesive to form an adhesive plug mechanically bonding the flexible base portion to the adhesive patch.

Additional and/or other aspects and advantages of the present invention will be set forth in the description that follows, or will be apparent from the description, or may be learned by practice of the invention.

### Brief Description of the Drawings

The above and/or other aspects and advantages of embodiments of the invention will be more readily appreciated from the following detailed description, taken in conjunction with the accompanying drawings, in which:
Fig. 1 is a top perspective view of an insulin infusion set having an introducer needle hub, a base assembly with an attached adhesive patch, and an introducer needle guard in accordance with an embodiment of the present invention;
Fig. 2 is a top perspective view of the insulin infusion set of Fig. 1 with its introducer needle hub and introducer needle guard removed, leaving only the base assembly;
Fig. 3 is a bottom perspective view of the infusion set base assembly of Fig. 2, illustrating the release liner and the flexible catheter that remains in the user's skin after the introducer needle is removed;
Fig. 4 is a partially exploded perspective view of the infusion set base assembly of Figs. 2 and 3, with the adhesive patch and release liner shown separately for clarity;
Fig. 5 is a side cross-sectional view of the rigid central portion of the infusion set base assembly of Figs. 2 and 3 that is formed during the first molding shot of a two-shot injection molding process;
Fig. 6 is a top perspective view of the infusion set base assembly and adhesive patch of Fig. 1;
Fig. 7 is a partial cross sectional view of the infusion set base assembly and adhesive patch of Fig. 1; and
Fig. 8 is a flowchart illustrating steps of a method of manufacturing a medical device in accordance with an embodiment of the present invention.

### Detailed Description of Embodiments of the Present Invention

Reference will now be made in detail to embodiments of the present invention, which are illustrated in the accompanying drawings, wherein like reference numerals refer to like elements throughout. The embodiments described herein exemplify, but do not limit, the present invention by referring to the drawings.

It will be understood by one skilled in the art that this disclosure is not limited in its application to the details of construction and the arrangement of components set forth in the following description or illustrated in the drawings. The embodiments herein are capable of other embodiments, and capable of being practiced or carried out in various ways. Phraseology and terminology used herein is for the purpose of description and should not be regarded as limiting. The use of "including," "comprising," or "having" and variations thereof herein is meant to encompass the items listed thereafter and equivalents thereof as well as additional items. Unless limited otherwise, the terms "connected," "coupled," and "mounted," and variations thereof herein are used broadly and encompass direct and indirect connections, couplings, and mountings. In addition, the terms "connected" and "coupled"" and variations thereof are not restricted to physical or mechanical connections or couplings. Further, terms such as "up," "down," "bottom," "top," "front," "rear," "upper," "lower," "upwardly," "downwardly," and other orientational descriptors are intended to facilitate the description of the exemplary embodiments of the present invention, and are not intended to limit the structure of the exemplary embodiments of the present invention to any particular position or orientation. Terms of degree, such as "substantially" or "approximately" are understood by those of ordinary skill to refer to reasonable ranges around and including the given value, for example, general tolerances associated with manufacturing, assembly, and use of the described embodiments.

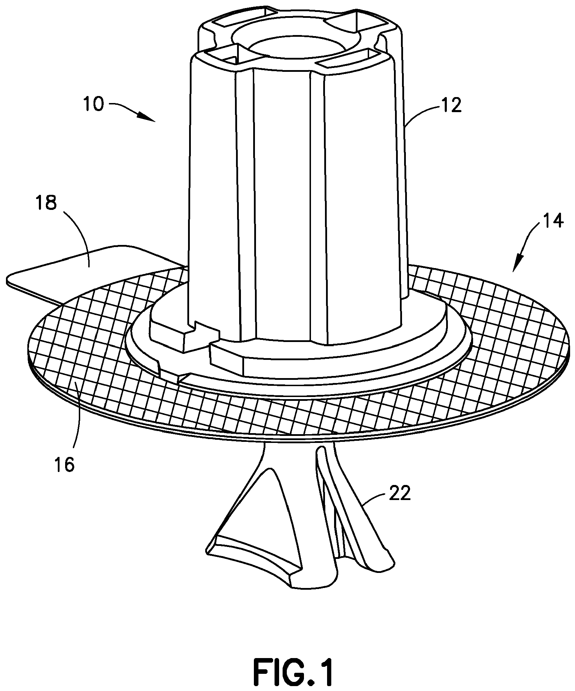

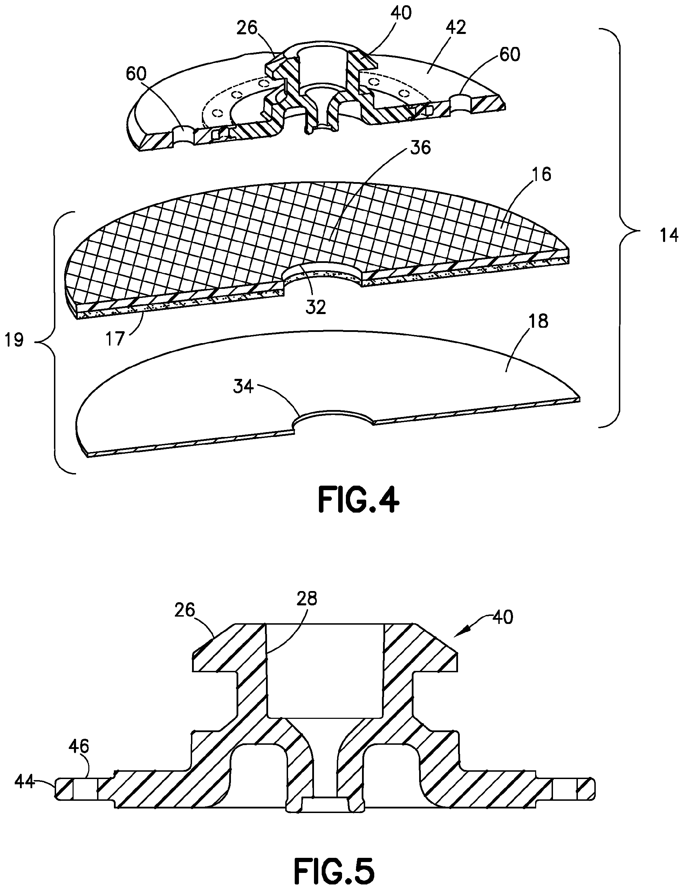

Fig. 1 illustrates an insulin infusion set 10 in accordance with an embodiment of the present invention. The infusion set comprises an introducer needle hub 12 engaged with a base assembly 14. The introducer needle hub 12 also serves as a passive telescopic shield for the introducer needle (not shown) after it is removed. The base assembly 14 includes a flexible patch 16 made of a flexible fibrous, textured, perforated or porous material such as nonwoven medical tape. The patch has an adhesive 17 (visible in Fig. 4) on its underside, which is used to secure the base assembly 14 to the user's skin. A removable backing layer or release liner 18 covers the skin-contacting adhesive 17 prior to use. The patch 16, adhesive 17 and release liner 18 constitute an adhesive patch assembly 19 as shown in Fig. 4.

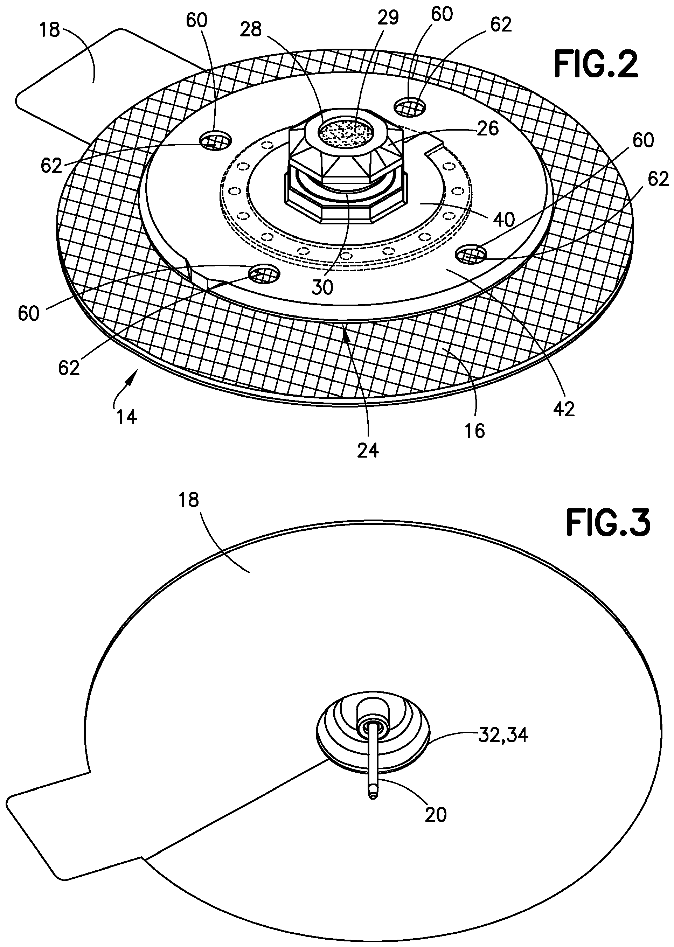

Fig. 1 illustrates a state in which the introducer needle hub 12 and base assembly 14 are ready to facilitate insertion of a flexible cannula or catheter 20 (visible in Fig. 3) and an introducer needle (not shown) into the user. A removable needle guard 22 covers the catheter 20 and the introducer needle (the latter initially received within and protruding from the catheter 20) prior to use.

Figs. 2 and 3 illustrate the infusion set 10 with the introducer needle hub 12 and needle guard 22 both removed. With the further removal of the release liner 18 and the with the catheter 20 penetrating into the subcutaneous skin layer, this is how the infusion set 10 would appear when adhered to the user's skin at an infusion site.

As shown in Fig. 2, the base assembly 14 includes a plastic central portion 24 that overlies the flexible adhesive patch 16 and is secured thereto by an adhesive on the upper surface of the patch. The plastic central portion 24 includes an upwardly facing fluid connector 26 having an opening 28 through which insulin is delivered through a pre-slit resilient septum 29 from a compatible fluid coupling (not shown) connected by flexible tubing to an infusion pump (both also not shown). The faceted shape of the fluid connector 26 and the recessed area 30 below its upper face facilitate latching connection with the fluid coupling at discrete rotational positions, allowing the user to position the flexible tubing as desired.

The adhesive patch 16 and release liner 18 are formed with two sets of identical, aligned holes 32, 34 and 36, 38 as shown more clearly in Fig. 4. The holes 32, 34 provide clearance for the emergence of the flexible catheter 20 as shown in Fig. 3.

As shown in Fig. 2 and in more detail in Figs. 4 and 5 (in which the catheter 20 and septum 29 are omitted for clarity), the plastic central portion 24 of the base assembly 14 is made up of two connected parts, a rigid inner hub or rigid base portion 40 defining the fluid connector 26 and a somewhat more flexible outer disk or flexible base portion 42. Both portions 40, 42 are generally circular in shape when viewed from above. The outermost rim 44 of the rigid inner hub 40 has a reduced thickness in which a plurality of through-holes 46 are formed. The through-holes 46 and the reduced thickness of the rim 44 facilitate bonding between the rigid inner portion 40 and the flexible outer disk 42.

More specifically, the rigid inner hub 40 is injection-molded in a first shot, and the flexible outer disk 42 is molded in a second shot during which the molten plastic material of the outer disk 42 flows around the rim 44 and through the holes 46. The shape of the rim 44 provides a double overlapping joint with the material of the flexible outer disk 42, and the holes 46 provide additional bonding surface area and additional mechanical interlocking.

The flexible outer disk 42 provides improved comfort and mobility of the infusion set base assembly 14 because it moves with the user during physical activity while minimizing contact of the rigid inner hub 40 with the user.

In embodiments of the invention, the patch assembly 19 can be die-cut from commercially available 3M™ Medical Nonwoven Tape (Product No. 1776). In this product, the patch material 16 is a ∼11.5 mil (0.3 mm) thick white spunlace nonwoven tape made of randomly oriented polyester fibers, the skin-contact adhesive 17 is an acrylate adhesive developed for medical/surgical use, and the release liner 18 is 83 lb. poly-coated Kraft paper of 6 mil (0.15 mm) thickness with a silicone release layer on both sides. Another suitable material is 3M™ Single Coated, Extended Wear Nonwoven Medical Tape (Product No. 4076). Still other materials that can be used are various single-coated medical tapes that are available from the Medical Materials and Technologies division of 3M Company. Other types of fibrous, textured, perforated or porous materials that are able to withstand the heat and pressure of the injection molding process can also be used for the adhesive patch 16 in the practice of the present invention.

In embodiments of the invention, the rigid inner hub 40 and flexible outer disk 42 may be made of any plastic materials that are suitable for injection molding. The rigid inner hub 40 is preferably made from a polyester blended material or polycarbonate, although this is not required. The flexible outer disk 42 is preferably made from a thermoplastic elastic material, some examples of which are listed in the table below Materials that allow for molding of the flexible outer disk 42 at lower temperatures are preferred to avoid adverse effects on the skin contact adhesive 17 provided between the patch 16 and the release liner 18.

| # | Material Type | Material Manufacturer | Material # |
|---|---|---|---|
| 1 | TPU | Elastocon | STK-040 |
| 2 | TPE | GLS | Versaflex OM3060 |
| 3 | TPE | GLS | Dynaflex G2711-1000-0 Nat'l |
| 4 | TPE | GLS | OM1040X-1 Nat'l |
| 5 | TPE | GLS | Versaflex HC2110-35N Nat'l |
| 6 | TPE | Tekniplex | Cellene MC2245 |
| 7 | Polyolefin Elastomer | Dow | Engage 8407 |
| 8 | Olefin Block Copolymer | Dow | Infuse 9807 |
| 9 | TPE | Teknor Apex | Medalist MD-46055 NAT XRD1 |

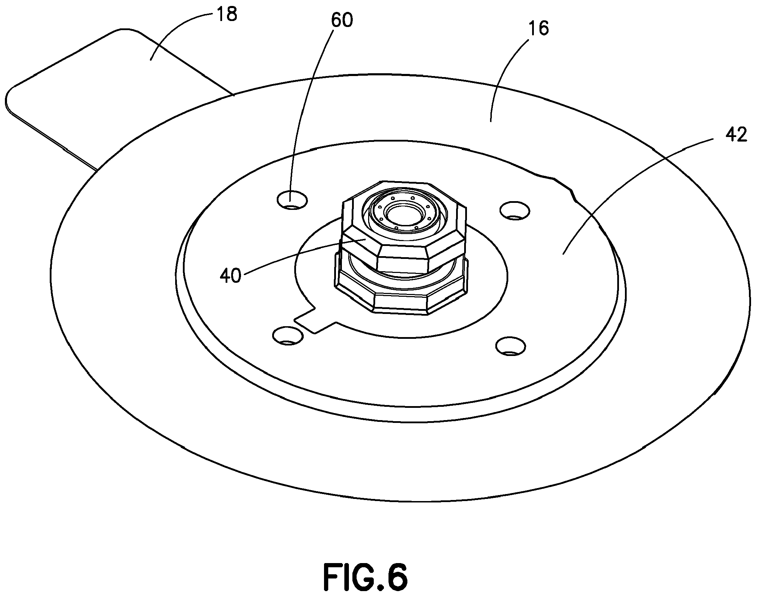

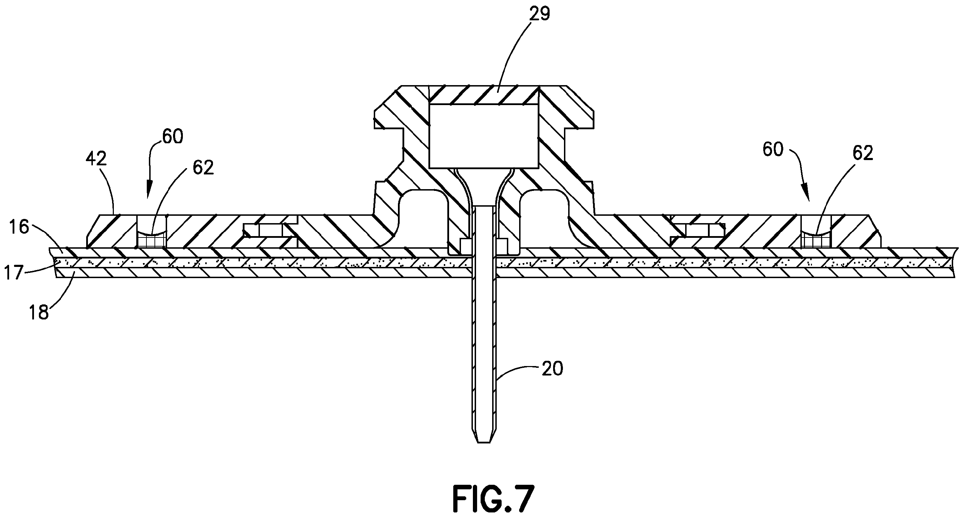

As previously noted, TPU and some other flexible materials sometimes have difficulty adhering to adhesive patches or pads such as adhesive patch 16 using only an adhesive. To solve this problem, in embodiments of the present invention, the flexible outer disk 42 is formed with one or more through-holes 60, as best shown in Figs 6 and 7. Preferably, the through-holes 60 are substantially vertical, or are disposed through the flexible outer disk 42 at approximately 90 degrees to both a top and a bottom surface of the flexible outer disk 42. Also preferably, the flexible outer disk 42 is formed with at least four vertical through-holes 60 arrayed evenly about the flexible outer disk about every 90 degrees, and most preferably, the flexible outer disk 42 is formed with six vertical through-holes 60 arrayed evenly about the flexible outer disk about every 60 degrees.

According to one embodiment, the patch assembly 19 is simply placed in contact with the flexible outer disk 42 and the rigid inner hub 40 by the manufacturer. Preferably, however, the patch assembly 19 is adhered to the flexible outer disk 42 and the rigid inner hub 40 by the manufacturer using a patch adhesive, such as 3M 1522 Double-Coated Adhesive. At this stage, the one or more through-holes 60 are in communication with the top or non-patient side of the adhesive patch 16. Although the through-holes 60 are preferably substantially vertical, and cylindrical, the through-holes 60 can have any shape and angle with respect to the top and bottom surfaces of the outer flexible disk 42 so long as the manufacturer can communicate with the non-patient side of the adhesive patch 16 therethrough.

Adhesive or glue, such as Dymax 1405M or Loctite 3977 visible light cure adhesive, is dispensed by the manufacturer into the through-holes 60 onto the adhesive patch 16 and the walls of the outer flexible disk 42 surrounding the through-holes 60. As shown in Fig. 7, the glue then cures to form an adhesive plug or mechanical locking feature 62 to mechanically bond the outer flexible disk 42 to the adhesive patch 16. Preferably, the glue is dispensed by a glue jet or pico jetting device.

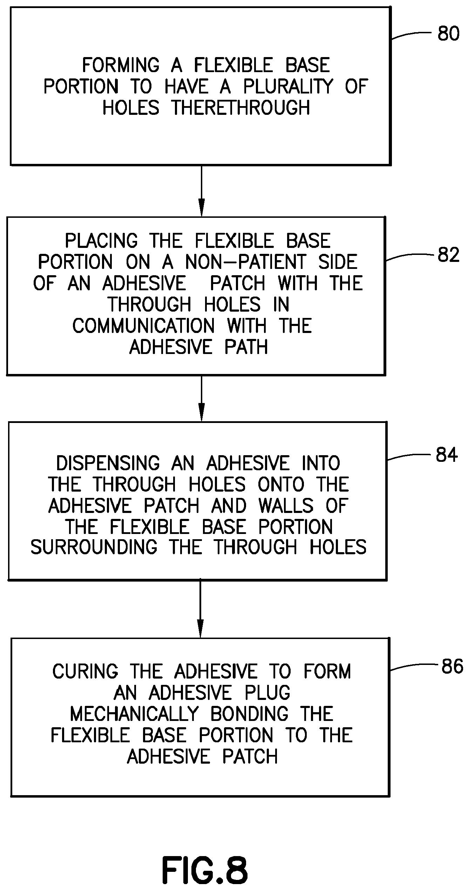

Fig. 8 is a flowchart illustrating steps of a method of manufacturing a medical device in accordance with an embodiment of the present invention. In operation 80, the manufacturer forms a flexible base portion to have a plurality of through-holes therethrough. Subsequently, in operation 82, the manufacturer places the flexible base portion on a non-patient side of an adhesive patch with the through-holes in communication with the adhesive patch. In operation 84, the manufacturer dispenses an adhesive into the through-holes onto the adhesive patch and walls of the flexible base portion surrounding the through-holes. And in operation 86, the manufacturer cures the adhesive to form an adhesive plug mechanically bonding the flexible base portion to the adhesive patch.

Although only a few embodiments of the present invention have been shown and described, the present invention is not limited to the described embodiments. Instead, it will be appreciated by those skilled in the art that changes may be made to these embodiments without departing from the principles and spirit of the invention. Any of the embodiments and/or elements disclosed herein may be combined with one another to form various additional embodiments not specifically disclosed, as long as they do not contradict each other. In other words, various aspects of the multiple embodiments may be employed independently or in combinations thereof. It is particularly noted that those skilled in the art can readily combine the various technical aspects of the various elements of the various exemplary embodiments that have been described above in numerous other ways, all of which are considered to be within the scope of the invention, which is defined by the appended claims and their equivalents.

## Claims

1. A method of manufacturing a medical device:
forming a flexible base portion to have a plurality of through-holes therethrough;
placing the flexible base portion on a non-patient side of an adhesive patch with the through-holes in communication with the adhesive patch;
dispensing an adhesive into the through-holes onto the adhesive patch and walls of the flexible base portion surrounding the through-holes; and
curing the adhesive to form an adhesive plug mechanically bonding the flexible base portion to the adhesive patch.

2. The method according to claim 1, wherein the flexible base portion comprises thermoplastic polyurethane.

3. The method according to claim 1, wherein forming the flexible base portion comprises injection molding the flexible base portion.

4. The method according to claim 3, wherein forming the flexible base portion comprises injection molding the flexible base portion onto a rigid base portion in a second shot of a two-shot molding process.

5. The method according to claim 1, wherein dispensing the adhesive comprises pico jetting the adhesive into the through-holes onto the adhesive patch and the walls of the flexible base portion surrounding the through-holes

6. An on-body medical device, comprising:
a flexible base portion having one or more through-holes therein;
an adhesive patch, the flexible base portion being disposed on a non-patient side of the adhesive patch with the through-holes in communication with the adhesive patch; and
an adhesive plug cured in the one or more through-holes on the adhesive patch and walls of the flexible base portion laterally surrounding the one or more through-holes, the adhesive plug mechanically bonding the flexible base portion to the adhesive patch.

7. The device according to claim 6, wherein the flexible base portion comprises thermoplastic polyurethane.

8. The device according to claim 6, wherein the flexible base portion is molded onto a rigid base portion.

9. The device according to claim 8, wherein said second portion has a lower durometer than said first portion.

10. The device according to claim 8, wherein flexible base portion is molded to laterally surround the rigid base portion.

11. The device according to claim 8, wherein the device further comprises a cannula for delivering a liquid medicament into a patient's skin, the cannula extending from the rigid portion through a hole in the adhesive patch.

12. The device according to claim 6, further comprising a release liner.
